# EUROPÄISCHE PATENTANMELDUNG

(11) **EP 3 338 772 A1**
(43) Veröffentlichungstag der Anmeldung: **27.06.2018**
(21) Anmeldenummer: 16206301.0
(22) Anmeldetag: 22.12.2016
(51) Int. Cl.: A61K 31/19, A61K 31/191, A61K 33/06, C07F 3/02, A23L 33/165

(54) **HYDROXYCARBONSÄURE-STABILISIERTE TRIMAGNESIUMDICITRATLÖSUNGEN, DEREN HERSTELLUNG UND HOCHKONZENTRIERTE MAGNESIUMLÖSUNGEN**

(71) Anmelder: Merz Pharma GmbH & Co. KGaA, 60318 Frankfurt Main (DE)
(72) Erfinder: BERLEKAMP, Uwe, 48432 Rheine (DE); ERBE, Thorsten, 60435 Frankfurt (DE); HOENNSCHEIDT, Christoph, 55130 Mainz (DE)
(74) Vertreter: Müller, Claudia

(57) **Zusammenfassung**

Die vorliegende Erfindung betrifft ein einfaches Verfahren zur *in situ* Herstellung von hoch konzentrierten Magnesiumlösungen. Durch direkte Umwandlung einer vor allem anorganischen Magnesiumverbindung wie Magnesiumoxid zu wasserlöslichem Magnesiumcitrat und anschließende Stabilisierung mit Hydroxycarbonsäuren werden stark wasserlösliche, hydratisierte Formen einer Hydroxycarbonsäure-stabilisierten Trimagnesiumdicitratlösung gebildet. In entsprechenden Produkten eingesetzt, können so Magnesiumlösungen mit einer insbesondere höheren Konzentration als bisher (also ≥ 20g(Mg²⁺) L⁻¹) an reinem Magnesium erhalten werden. Derartige Zubereitungen können als solche oder in Form von Nahrungsergänzungsmitteln eingesetzt werden.

## Beschreibung

### Gegenstand der Erfindung

Die vorliegende Erfindung betrifft ein einfaches Verfahren zur *in situ* Herstellung von hoch konzentrierten Magnesiumlösungen. Durch direkte Umwandlung einer vor allem anorganischen Magnesium(ausgangs)-verbindung wie Magnesiumoxid zu wasserlöslichem Magnesiumcitrat und anschließende Stabilisierung mit Hydroxycarbonsäuren, insbesondere Milchsäure, Äpfelsäure oder Mischungen hiervon, werden stark wasserlösliche, hydratisierte Formen eines Hydroxycarbonsäure-stabilisierten Trimagnesiumdicitrates (in Lösung) gebildet. In entsprechenden Produkten eingesetzt, können so Magnesiumlösungen mit einer insbesondere höheren Konzentration als bisher, also ≥ 20 g(Mg²⁺)L⁻¹, an reinem Magnesium erhalten werden. Die Erfindung betrifft daher auch Produkte oder Zubereitungen aus Wasser und Hydroxycarbonsäure- stabilisierten Trimagesiumdicitraten (Magnesiumcitratlösungen). Derartige Zubereitungen oder Produkte können als solche oder in Form von supplementierten Lebensmitteln eingesetzt werden.

Die Erfindung betrifft daher auch die Herstellung von derartigen Lebensmitteln wie Nahrungsergänzungsmitteln oder auch von pharmazeutischen Zubereitungen wie Medizinprodukten oder Arzneimitteln unter Verwendung der beschriebenen Hydroxycarbonsäure-stabilisierten Trimagnesiumdicitrate bzw. Lösungen hiervon.

### Hintergrund

Calcium und Magnesium bzw. deren Salze zählen zu den essentiellen Mineralien des tierischen bzw. menschlichen Organismus, die diesem daher in jeweils ausreichender Menge zur Verfügung gestellt werden sollten, insbesondere bei erhöhtem Bedarf. Während Calcium vor allem für den Aufbau der Knochen und für die Blutgerinnung ein entscheidender Faktor ist, ist Magnesium insbesondere für die Skelettmuskulatur, das Nervensystem und die Herzfunktion essentiell. Weiterhin ist Magnesium, aufgrund seiner Mitwirkung bei mindestens 300 Enzymsystemen, zur Energiebereitstellung im Organismus von großer Bedeutung.

Die Bereitstellung von Präparaten und Zubereitungen, welche geeignete Mengen an diesen Mineralien aufweisen, umfasst daher nicht nur die Aufgabe der galenischen Umsetzung, sondern vor allem auch die Aufgabe, eine ausreichende Bioverfügbarkeit im Organismus zu ermöglichen.

Von beiden Mineralien existieren eine Reihe verschiedener organischer und anorganischer Verbindungen mit jeweils unterschiedlichen Eigenschaften. Im Allgemeinen sind anorganische Salze in Wasser weniger löslich als organische, letztere können aber aus organoleptischen Gründen zu Problemen führen.

So ist Calciumgluconat zwar gut löslich, neigt aber bei erhöhter Temperatur bei entsprechenden Herstellungsprozessen zu Verfärbungen (EP 1711072 B1, Abs. 0009). Anorganische Magnesiumsalze wie das Oxid, Hydroxid, Carbonat, Chlorid sind im Allgemeinen weniger löslich als organische Verbindungen wie das Lactat, Citrat, Aspartat/-Hydrochlorid, allerdings weist Magnesiumlactat einen bitteren Geschmack auf, während Magnesiumcitrat eher sauer ist.

Ferner ist auch die hydratisierte Form von Salzen von Bedeutung für deren Löslichkeit:
So beschreiben Apelbat et al. in J. Chem. Thermodynamics 25 (1993) 1443-1445 die Änderung des Löslichkeitsproduktes von Magnesiumcitrat in Abhängigkeit seines Hydratisierungsgrades wie folgt:
   - Trimagnesiumdicitrat*Nonahydrat (C₁₂H₁₀O₁₄Mg₃*9H₂O): 18,14 g(Mg²⁺)kg⁻¹_{H2O} (25 °C)
   - Trimagnesiumdicitrat*Tetradecanhydrat (C₁₂H₁₀O₁₄Mg₃*14H₂O): 19,40 g(Mg²⁺)kg⁻¹_{H2O} (22 °C)

Daraus ergibt sich, dass je nach Hydratisierungs- und/oder Koordinationsstatus eine unterschiedliche Löslichkeit vorliegen kann. Mit der irreversiblen Substitution stabilisierender Hydratverbindungen am Magnesium-Ion durch potentiell wechselwirkende Partner sinkt also das Löslichkeitsprodukt bei Wiederauflösen in Wasser rapide. Dieser Fall tritt insbesondere dann ein, wenn z.B. im Zuge eines Herstellungsverfahrens ein Trocknungsschritt integriert ist. Bei der Herstellung einer Zubereitung zur Versorgung eines Organismus mit einer Menge an Magnesium ist daher neben den galenischen Anforderungen wie z.B. hinsichtlich des Geschmacks und der Farbe auch auf die Stabilität bzw. die Löslichkeit der eingesetzten Verbindungen zu achten, um eine ausreichende Bioverfügbarkeit zu gewährleisten.
Derzeit sind Dosiereinheiten von maximal 300 mg Magnesium in einem Volumen von mindestens 25 mL verfügbar, was einer Konzentration von 12 g(Mg²⁺) L⁻¹ an reinem Magnesium entspricht.

### Stand der Technik

In der WO2005/072539 (EP B 1 711 072 B1) wird ein Verfahren zur Herstellung einer sehr gut löslichen Calciumverbindung aus Lactat und Malat in bestimmten molaren Verhältnissen beschrieben. Dazu werden anorganische Salze wie Calciumoxid, Calciumhydroxid in Wasser, vorzugsweise in der Wärme gelöst und mit definierten Mengen einer Mischung von Milchsäure und Äpfelsäure versetzt. In einem abschließenden Trocknungsschritt werden nach Entfernen des Wassers geschmacklich angenehme Calciumverbindungen mit ca. 18% Calciumanteil erhalten. Diese lassen sich sehr gut zur Anreicherung in Lebensmitteln verwenden, da durch die sehr gute Löslichkeit z.B. in 200 ml Wasser (4 g) die fünffache Menge an RDA-Calcium gelöst werden kann (anstelle der zuvor nur dreifachen Menge mit Calciumlactat, siehe WO 200507-2539, Seite 11).

Nach der EP 1 217 904 B1 werden Getränke und andere flüssige, mit einem Calcium-Magnesium-Lactat-Citrat-Komplex angereicherte Lebensmittel beschrieben. In diesem Komplex liegt Calcium in einem mindestens dreifachen Überschuss gegenüber Magnesium vor. Das Verhältnis von Lactat und Citrat zum Erdalkalikomplex beträgt 0,2 bis 20 bzw. umgekehrt. Die Komplexverbindung wird insbesondere aus alkalischen Calcium- und Magnesiumsalzen wie Oxid, Hydroxid unter Zugabe einer Lösung von Citronensäure und Milchsäure analog der oben beschriebenen Methode für Calciumkomplexe hergestellt, hier jedoch vor allem durch Stabilisierung mit Kohlenhydraten wie Fructose, Saccharose, Glucose (siehe Ansprüche 1-6). Die Löslichkeit in Wasser wird mit 900 mg/L Calcium und 300 mg/L Magnesium (siehe Beispiel 2) angegeben.

Gemäß der US 2015/0224137 A1 werden Citrat angereicherte sprudelnde Calcium-Magnesium-Nährstoffmittel erhalten durch Mischung von pulverisiertem Calciumcarbonat, Magnesiumcitrat und Citronensäure (im Überschuss von mindestens 10 Milliäquivalente), wobei pro Dosiereinheit 175 mg Magnesium erhalten werden soll (siehe Seite 3, Abs.0035, 0036). Mit derartigen Verbindungen soll eine schnelle Verfügbarkeit, insbesondere von Calcium (vor allem bei Herzanfällen), trotz Defekten bei Magensäuresekretionen ermöglicht werden. Die RU 2402241 C1 beschreibt die Herstellung einer Magnesiumverbindung speziell für diätetische Lebensmittel. Dazu wird laut Abstract eine 22,2 bis 33,3 %-ige wässrige Lactatlösung mit Magnesiumoxid bei 70° C umgesetzt, bis der pH-Wert der Lösung 6,5 bis 7,2 beträgt und sich Magnesiumlactat-Kristalle bilden. Diese werden extrahiert, kristallisiert und getrocknet. Aufgrund ihrer Reinheit können sie in diätetischen Lebensmitteln eingesetzt werden. Eine Löslichkeit oder Bioverfügbarkeitswerte hiervon sind nicht angegeben.

In der EP 1 762 231 B1 werden Magnesium-Mikrotabletten (MG-Verla) beschrieben, wobei die Bioverfügbarkeit durch eine retardierte Freisetzung erfolgen soll. Dazu wird eine Matrix aus einer Magnesiumverbindung mit einem Retardlack überzogen. Als Magnesiumverbindung eignen sich alle bekannten Derivate wie Oxid, Hydroxid, Citrat, Aspartat und insbesondere Magnesium-L-Aspartat-Hydrochlorid.

Gemäß der WO 2009/147667 A1 (EP 2 285 229 B1) werden in Lebensmitteln verwendbare Kalium-Magnesium-Citrat-Verbindungen beschrieben. Es wird postuliert, dass durch Erhöhung des Verhältnisses von Magnesium zu Kalium auf 1:1 bis 2,5:1 die Magnesiumaufnahme effektiver sein soll (siehe Seite 2, Mitte). Dazu wird z.B. eine 0,25 bis 1,2 M wässrige Citratlösung bei Temperaturen unter 25° C mit 2-7 Mol einer Magnesiumverbindung (z.B. Magnesiumhydroxid) und 2,5 bis 3 Mol einer Kaliumverbindung (z.B. Kaliumhydroxid) versetzt. Solche Produkte sollen bei Einsatz von 3 g in einem Liter Sojamilch nach 2 Tagen zu einer stabilen Lösung mit 445 mg/L bzw. 545 mg/L Magnesium führen. Hingegen wurden von 2,2 g herkömmlichem Tri-Magnesiumcitrat-Nonahydrat in 1 Liter Sojamilch nach 2 Tagen nur noch 307 mg/L erhalten. In den beschriebenen Kalium-Magnesiumzubereitungen war demnach der Magnesiumgehalt stabiler.

Gemäß der DE 38 32 638 A1 werden Magnesiumverbindungen mit besonders guten organoleptischen Eigenschaften als Zusatz für Nährstoffe, Arzneimittel und Nahrungsmittel beschrieben. Diese werden erhalten durch Mischen/Granulieren von trockenem Magnesiumlactat (z.B. 2.954 g) und pulverisiertem saurem zweiwertigem Magnesiumcitrat (1.750 g), Granulierflüssigkeit (520 g) und Trocknen bei 55°C/24 h. Welche tatsächliche Löslichkeit bzw. Bioverfügbarkeit an Magnesium hiervon vorliegt, ist nicht beschrieben.

Mit den oben beschriebenen Zubereitungen bzw. Herstellungsverfahren werden jedoch noch keine Produkte mit Magnesium als hauptsächlichem Mineral offenbart, welche alle Anforderungen an derartige Produkte wie Geschmack, Stabilität, Wasserlöslichkeit, Bioverfügbarkeit in mindestens RDA-angemessenen Mengen aufweisen und dabei auf einfache Weise erhalten werden können.

### Aufgabe

Die Aufgabe der vorliegenden Erfindung ist es, eine Magnesiumverbindung bzw. Zubereitung bereitzustellen, welche sowohl den allgemeinen Anforderungen im Zusammenhang mit Nahrungsmitteln, Nahrungsergänzungsmitteln oder auch Arzneimitteln entsprechen, als auch ohne andere Mineralien/Stabilisatoren stabil bleibt. Ferner soll eine hohe Bioverfügbarkeit derart ermöglicht werden, dass die Menge an verabreichtem Produkt reduziert werden kann, so dass ein möglichst hohes Verhältnis von bioverfügbarem Wirkstoff zu verabreichter Gesamtproduktmenge erhalten werden kann. Darüber hinaus sollen derartige Magnesiumprodukte auf einfache und kostengünstige Weise herstellbar sein.

### Lösung

Diese Aufgabe wird erfindungsgemäß gelöst durch die Merkmale des Anspruchs 1 bzw. 7. Erfindungsgemäß werden Magnesiumverbindungen mit hoher Bioverfügbarkeit/Wasserlöslichkeit bereitgestellt, wenn sie als Hydroxycarbonsäure - stabilisierte Citrate vorliegen.

### Abbildung 1

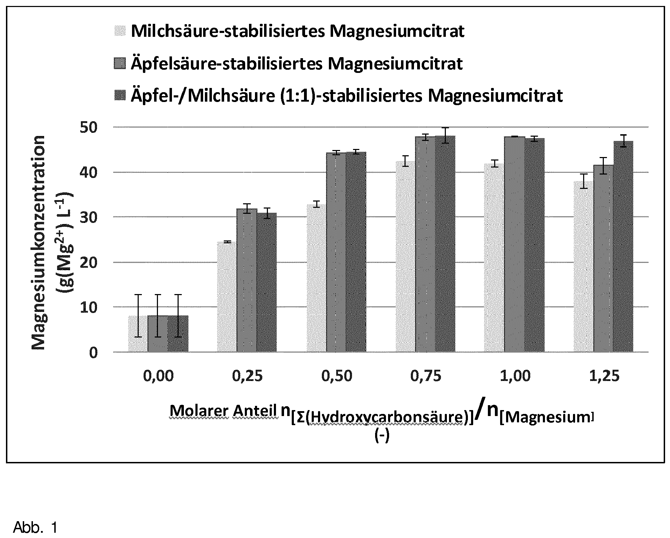

Abb. 1 stellt grafisch den Einfluss der Löslichkeit von Magnesiumcitrat in Gegenwart unterschiedlich eingesetzter molarer Mengen an Hydroxycarbonsäure (Milchsäure und Äpfelsäure) bzw. deren 1:1 Mischungen gegenüber Magnesium dar. Bei allen untersuchten Hydroxycarbonsäuren ist mit steigendem molarem Anteil bis zu einem Grenzwert eine Erhöhung der Magnesiumcitratlöslichkeit zu erkennen.

### Nähere Beschreibung der Erfindung

Erfindungsgemäße Produkte (stabilisiertes Magnesiumcitrat in einem Lösungsmittel) können erhalten werden durch folgende Vorgehensweise:
i) Lösen geeigneter Mengen an Citronensäure in einer ausreichenden Menge eines geeigneten Lösungsmittels, insbesondere Wasser, vor allem bei Temperaturen von 20° C bis 99 °C, vorzugsweise bei 50 °C bis 95 °C, insbesondere bei 70 °C bis 95 °C, und vor allem bei ca. 70 °C, und vorzugsweise unter Rühren;
ii) Zugabe einer molaren Menge, insbesondere 1,0 bis 2,0 Mol, vor allem 1,3 bis 1,7, insbesondere 1,5 (3/2) Mol, im Verhältnis zum molaren Anteil an Citronensäure, einer oder mehrerer, vorzugsweise einer, vor allem anorganischer Magnesium(ausgangs)-verbindungen wie Magnesiumsalz, insbesondere ausgewählt aus Magnesiumoxid, Magnesiumhydroxid, Magnesiumchlorid oder Magnesiumcarbonat, oder auch Mischungen hiervon;
iii) Zugabe einer in Bezug auf die Magnesium(ausgangs)-verbindung, wie oben angegeben, bestimmten molaren (stöchiometrischen) Menge an Hydroxycarbonsäure, ausgewählt wie nachfolgend beschrieben, vor allem Milchsäure, Äpfelsäure oder Mischungen hiervon, vorzugsweise unter Rühren und/oder vorzugsweise bei Raumtemperatur;
iv) Gewinnung des so erhaltenen Produktes mit einem hohen Gehalt an bioverfügbarem, gelösten Magnesium in an sich bekannter Weise, und ggf. Weiterverarbeitung zu gebrauchsfertigen Dosiereinheiten, wobei das Magnesium als Hydroxycarbonsäure-stabilisierte Magnesiumcitratverbindung der allgemeinen Formel

   Mgₓ(C₆H₅O₇)_{y} (HCS)_{z} (14-z) • H₂O

   vorliegt, worin x = 3, y = 2, z = 0,25 - 4,00, Mg = Magnesium-Ion, und (C₆H₅O₇)= Citrat-Ion, HCS Hydroxycarbonsäure bedeuten.

In Schritt ii) erfolgt die Umsetzung normalerweise solange, bis sich die Feststoffanteile gelöst haben. Falls die Zubereitung erwärmt worden ist, wird sie abgekühlt, bevor in Schritt iii) die Hydroxycarbonsäure hinzugefügt wird.

Nach Schritt iii) und vor Schritt iv) kann ggf. ein Hinzufügen von geeigneten/gewünschten, in derartigen Produkten möglichen, oder technologisch erforderlichen Hilfs- oder Zusatzstoffen wie nachfolgend beschrieben oder besonders bevorzugt ausgewählt aus Konservierungsmitteln, Emulgatoren, Süßstoffen, Geschmacks- und Aromastoffen oder Mischungen hiervon, erfolgen.
Die Umsetzung in Schritt i) kann bei gleicher oder anderer Temperatur als in Schritt ii) erfolgen. In einer weiteren Ausführungsform kann die Umsetzung in Schritt ii) bei einer Temperatur von 70 °C bis 95 °C erfolgen.
In einer weiteren bevorzugten Ausführungsform wird die Temperatur in der wässrigen Lösung (Verfahrensschritt i) auf ca. 50 °C bis ca. 97 °C, oder auch 60 bis 95 ° C, besonders bevorzugt auf ca. 85 °C bis ca. 95 °C, ganz besonders bevorzugt auf ca. 60 °C bis 80 °C erhöht.
Durch die Erhöhung der Temperatur wird die Lösegeschwindigkeit des Magnesiumsalzes, vor allem Magnesiumhydroxids, -oxids oder -carbonats und insofern die Gesamtmenge an lösbarem Magnesium in einer vorgegebenen Wassermenge deutlich erhöht.
In einer besonders bevorzugten Ausführungsform wird die Temperatur des Gemisches in Verfahrensschritt i) auf ca. 70°C eingestellt.

Der pH Wert der übersättigten, wässrigen Lösungen stellt sich bevorzugt auf pH 3 ein.

Ebenfalls von Vorteil ist es, wenn die Magnesium(ausgangs)-verbindung, insbesondere ein Magnesiumsalz wie oben beschrieben, in Schritt ii) in Form eines Pulvers hinzugefügt wird.

Bevorzugt wird die Hydroxycarbonsäure gemäß Schritt iii) ausgewählt aus Glykolsäure, Milchsäure, Äpfelsäure, Weinsäure oder auch Gluconsäure, Glucuronsäure, Glycerinsäure, Xylonsäure, Galacturonsäure, Iduronsäure, Mucinsäure, Glucarsäure Chinasäure, oder Mischungen hiervon.
Insbesondere bevorzugt sind hier als Hydroxycarbonsäure Milchsäure, Äpfelsäure oder Mischungen hiervon.

Die erfindungsgemäß erhaltene Hydroxycarbonsäure-stabilisierte Magnesiumcitratlösung hat insbesondere einen sehr hohen Magnesiumgehalt von ca. 2,0 bis 4,0 Gew.-% oder 16 g(Mg²⁺) L⁻¹ bis 45 g(Mg²⁺) L⁻¹, insbesondere ≥ 20 g(Mg²⁺) L⁻¹ bis 45 g(Mg²⁺) L⁻¹.

Anmeldungsgemäß können so über einen direkten in situ - Umwandlungsprozess einer Magnesium(ausgangs)verbindung, wie vor allem von Magnesiumoxid, zum wasserlöslichen Magnesiumcitrat und anschließender Stabilisierung durch Hydroxycarbonsäuren, stark wasserlösliche, hydratisierte Formen des stabilisierten Trimagnesiumdicitrats (Trimagnesium-Hydroxycarbonsäure-Hydrat-Verbindungen) gemäß Formel (I) gebildet und gleichermaßen aufrechterhalten werden. Die Umsetzung erfolgt z.B. gemäß nachfolgendem Schema 1.
Umsetzungsschema 1:

x• Mg²⁺_{(s, aq)} + y•C₆H₈O₇1•H₂O_{(aq)} + 13•H₂O_{(aq)} → Mgₓ(C₆H₅O₇)_{y(aq)}14•H₂O_{(aq)} + y3•H⁺_{(aq)} Mgₓ(C₆H₅O₇)_{y(aq)}14H₂O_{(aq)} + z•HCS_{(aq)} → Mgₓ(C₆H₅O₇)_{y}(HCS)_{z}*14-z)•H₂O_{(aq)} (I)

worin C₆H₈O₇ = Citronensäure, (C₆H₅O₇)³⁻ = Citrat und HCS = Hydroxycarbonsäure bedeuten. S bedeutet das Gegenion der Magnesium(ausgangs)-verbindung wie vor allem Salze. Diese sind vor allem ausgewählt aus Magnesiumoxid (MgO) mit S=O, -hydroxid (Mg(OH)₂) mit S=(OH⁻)₂, Magnesiumchlorid (MgCl₂) mit S= (Cl⁻)₂ oder -carbonat (MgCO₃) mit S=(CO₃)²⁻.

Weiterhin bedeuten x = 3, y = 2, z = 0,25 - 4,00, vorzugsweise 0,25 bis 3,75, oder auch 1,5 bis 3,75, insbesondere 1,5 bis 3,0, vor allem 2,25 bis 3,0. Z bedeutet die Gesamtmenge an allen eingesetzten Hydroxycarbonsäuren, welche insbesondere bei Mischungen unterschiedliche, voneinander verschiedene Werte darstellen und daher unabhängig voneinander angegeben werden kann. Beispielsweise kann die Kombination Milchsäure und Äpfelsäure angegeben werden als Milchsäure= Anteil (I) + Äpfelsäure = Anteil (m), woraus sich z = I + m ergibt. Angegeben sind jeweils die molaren Anteile.

Erfindungsgemäße Verbindungen weisen also die Formel (I) auf:
Mgₓ(C₆H₅O₇)_{y}(HCS)_{z}(14-z)•H₂O_{(aq)}, mit x = 3, y = 2, z = 0,25 - 4,00, vorzugsweise 0,25 bis 3,75, vor allem 0,25 bis 2,25.

Insbesondere können bei Zusatz einer weniger als äquimolaren Menge bis zu einer 1,6-molaren Menge, also von 0,75 bis 1,6, an Hydroxycarbonsäure oder Mischungen hiervon, bezogen auf die molare Menge des Magnesiums aus der Ausgangsverbindung (wie Oxid, Hydroxid, Carbonat u.ä.), hochkonzentrierte, stabile Magnesiumlösungen in Wasser im Bereich von bis zu 45 g(Mg²⁺) L⁻¹ bei Raumtemperatur und mehr erreicht werden, bevor eine Kristallisation einsetzt. Bei fehlender Hydroxycarbonsäure kann jedoch nur eine Lösung mit einer maximalen Magnesium-Konzentration von 19,40 g(Mg²⁺) kg⁻¹ (Wasser) bei Raumtemperatur als Trimagnesiumdicitrat*Tetradecanhydrat (C₁₂H₁₀O₁₄Mg₃*14H₂O) erzeugt werden (s.o.). Der Anstieg, aufgrund der anmeldungsgemäßen Produkte, ist unter den genannten Bedingungen somit etwa um den Faktor 2,3 größer.

Bevorzugte Verbindungen gemäß vorliegender Anmeldung sind folgende Hydroxycarbonsäure-stabilisierte Trimagnesiumdicitratverbindungen der allgemeinen Formel (I) gemäß der nachfolgenden Formeln II, III, IV:
Milchsäure-stabilisierte (C₃H₆O₃) Magnesiumcitratverbindung

   Mgₓ(C₆H₅O₇)_{y}(C₃H₆O₃)_{z}*(14-z)•H₂O (II)

   (x = 3, y = 2, z = 0,25 - 3,75)
Äpfelsäure-stabilisierte (C₄H₆O₅) Magnesiumcitratverbindung

   Mgₓ(C₆H₅O₇)_{y}(C₄H₆O₅)_{z}*(14-z)•H₂O (III)

   (x = 3, y = 2, z = 0,25 - 3,75)
Milch-/Äpfelsäure (1:1)-stabilisierte Magnesiumcitratverbindung

   Mgₓ(C₆H₅O₇)_{y}*[(C₃H₆O₃)ₗ(C₄H₆O₅)ₘ]_{z}*(14-z)•H₂O (IV)

   (x = 3, y = 2, z = l + m = 0,25 - 3,75)

Der molare Anteil der Hydroxycarbonsäure, z.B. der Milchsäure, liegt vor allem in einem Bereich von 0,5 bis 1,2 und der molare Anteil des Citrates liegt in einem Bereich von 0,66, jeweils bezogen auf den molaren Anteil an Magnesium.
Insbesondere zeigte sich, dass erfindungsgemäß Magnesiumcitratlösungen mit sehr hohem Anteil an Magnesium, insbesondere 450 mg(Mg²⁺)/Dosiereinheit, erhalten werden können, wenn in den Verbindungen mit der molaren Verhältnisformel Mgₓ(C₆H₅O₇)_{y}(Hydroxycarbonsäure)_{z}*(14-z)•H₂O mit x = 3, y = 2) und die entsprechenden molaren Anteile z der Hydroxycarbonsäure oder die molaren Anteile der Mischungen aus Hydroxycarbonsäuren, insbesondere der Mischungen aus Milchsäure und Äpfelsäure (z= m + I) bei 0,5 bis 1,6 liegen.

Erfindungsgemäß ist es so möglich, Magnesiumlösungen mit einer Konzentration von mehr als 20 g(Mg²⁺) L⁻¹ bei Raumtemperatur an reinem Magnesium bereitzustellen. Überraschender Weise wird also mit der Integration einer Hydroxycarbonsäure in ein Magnesiumcitrat eine stabile wasserlösliche Lösung gebildet, in der das Löslichkeitsprodukt signifikant erhöht ist.
Die anmeldungsgemäß beschriebene *in situ* Umsetzung führt demnach zum Erhalt wichtiger Hydratverbindungen an dem Magnesiumcitrat, wobei darüber hinaus ein weiterer Trocknungsschritt nicht erforderlich ist. Dies ist im Hinblick auf die Stabilität der Verbindung besonders vorteilhaft. Da die Erhöhung der Wasserlöslichkeit unmittelbar mit der Erhöhung der Bioverfügbarkeit korreliert, kann weiterhin eine Erhöhung der Dosierung insbesondere in flüssiger Darreichungsform erreicht werden.

Dies konnte im Hinblick auf vorbeschriebene ähnliche bzw. analoge für Calcium beschriebene Verfahren nicht erwartet werden, da Calcium eine weitaus höhere Komplexbildungskonstante als Magnesium aufweist, was eine Überführung in eine hochkonzentrierte Lösung chemisch einfacher macht.

Erfindungsgemäß wird somit das Problem gelöst, Magnesium in hoher Konzentration in Lösung zu bringen und es somit leicht bioverfügbar zu machen, da eine irreversible Substitution stabilisierender Hydratverbindungen am Magnesium-Ion durch potentiell wechselwirkende Partner, wie dies bei durch einen Trocknungsschritt erhaltenen Produkten z.B. bei Calcium-Produkten der Fall ist, vermieden wird. Daher konnte das bzgl. der beschriebenen Calciumverbindungen bekannte Verfahren hier nicht zum Ziel führen und auch keinen Hinweis auf die unterschiedliche Verfahrensweise geben. Denn, anders als erfindungsgemäß, wird im Calcium- bzw. auch im bekannten Calcium-Magnesium-Verfahren eine Mischung aus Hydroxycarbonsäuren eingesetzt, bzw. der Hydratwasseranteil wird durch einen geeigneten Trocknungsschritt entfernt. Es war insofern überraschend, dass auch ohne Trocknungsschritt, jedoch unter anfänglicher Bildung eines Citratkomplexes, ein leicht handhabbares Produkt erhalten werden kann, welches eine hohe Bioverfügbarkeit an Magnesium, ohne Calcium, aufweist.

### Erläuterung der Erfindung gemäß Abbildung 1

Die Erfindung wird anhand der Abbildung 1 entsprechend den Ergebnissen der Tabelle 1 gemäß Beispiel 3 der Anmeldung näher erläutert:
Durch Gewinnung einer anmeldungsgemäßen Verbindung (I) aus Magnesiumoxid und Citronensäure sowie nachfolgender Zugabe von Milchsäure, Äpfelsäure oder Mischungen hiervon (wie in Beispiel 1 und 2 beschrieben) werden Lösungen erhalten, wobei unterschiedliche Löslichkeitswerte des Magnesiums wie in Abbildung 1 bzw. Tabelle 1 dargestellt erzielt werden. Hieraus ist Folgendes ersichtlich:
   Die maximal erreichbare Löslichkeit in der Milchsäure-stabilisierten Trimagnesium-dicitratverbindung ist bei einem molaren Anteil von Milchsäure gegenüber Magnesium bei 0,75 (entspricht bei n(Mg²⁺) = 3 mol, n (Milchsäure) = 2,25, It. Tabelle 1) festzustellen. Hier erreicht die Magnesiumcitratlöslichkeit einen maximalen Wert von 42,53 ± 1,17 g(Mg²⁺) L⁻¹ (siehe Tabelle 1). Die Löslichkeit sinkt bei größeren Anteilen an Milchsäure wieder, bleibt aber in weiten Bereichen (bis zu einem molaren Anteil von 1,25) noch immer über dem bisher im Stand der Technik erreichten Wert (37,96 ± 1,52 g(Mg²⁺) L⁻¹) Mit der Äpfelsäure-stabilisierten Trimagnesium-dicitratverbindung ist eine maximale Löslichkeit von 47,92 ± 0,07 g(Mg²⁺) L⁻¹ bei molarem Anteil zwischen 0,75 bis 1,00 (entspricht bei n(Mg²⁺) = 3 mol, n(Äpfelsäure) = 2,25 bis 3,0, It. Tabelle 1) zu erkennen. Die Löslichkeit sinkt auch hier bei steigendem molaren Anteil der Äpfelsäure gegenüber dem Magnesium, bleibt aber auch hier (bis zu einem molaren Anteil von 1,25) immer noch über dem bekannten Wert (41,44 ± 1,93 g(Mg²⁺) L⁻¹.

Mit einer 1:1 Mischung an Milchsäure und Äpfelsäure (jeweils ein molarer Anteil von 0,375) lässt sich Magnesiumcitrat ebenso stabilisieren. Hier ist bei einem molaren Gesamtanteil beider Hydroxycarbonsäuren eine maximale Löslichkeit von 48,12 ± 1,75 g(Mg²⁺) L⁻¹ bei einem molaren Anteil gegenüber Magnesium mit 0,75 festzustellen. Die Löslichkeit bleibt hier bei weiterer Zugabe der Hydroxycarbonsäure-Mischung nahezu konstant gleich.

### Verwendete Komponenten

### 1. Magnesium(ausgangs)-verbindung

Geeignete Magnesium(ausgangs)verbindungen sind insbesondere Salze, vor allem Magnesiumoxid, Magnesiumcarbonat, Magnesiumhydroxid, Magnesiumchlorid. Besonders bevorzugt ist Magnesiumoxid oder auch Magnesiumcarbonat, oder auch Mischungen hiervon.

### 2. Säuren:

Geeignete Hydroxycarbonsäuren, welche als stabilisierendes Agens erfindungsgemäß eingesetzt werden können, sind Glykolsäure, Milchsäure, Äpfelsäure, Weinsäure oder auch Gluconsäure, Glucuronsäure, Glycerinsäure, Xylonsäure, Galacturonsäure, Iduronsäure, Mucinsäure, Glucarsäure und Chinasäure.

Besonders bevorzugt sind Milchsäure, Äpfelsäure oder Mischungen hiervon in molaren Anteilen bzw. Gesamtanteilen, bezogen auf die Magnesiumstoffmenge wie angegeben, vor allem 0,8 bis 2,0 mol, insbesondere in 1,0 bis 1,75, vor allem 1,15 bis 1,65 molaren Menge, bezogen auf Magnesium.

Milchsäure wird bevorzugt als Flüssigkeit wie z.B. 80-90 %-ig in Wasser (Milchsäureracemat) eingesetzt, Äpfelsäure wird bevorzugt als Feststoff eingesetzt.

### 3. Citratverbindung

Als Citrat liefernde Verbindung wird insbesondere Citronensäurepulver gewählt.

### 4. Lösungsmittel

Das Lösungsmittel ist vor allem gereinigtes Wasser.

### 5. Zusatzstoffe/Hilfsstoffe

Geeignete Zusatzstoffe können je nach galenischen Anforderungen unterschiedlich ausgewählt werden. Die Gruppe an Zusatzstoffen umfasst vor allem Konservierungsmittel wie Sorbinsäure, Benzoesäure oder deren Salze, Farbstoffe, Vitamine, Aromen/Geschmacksstoffe, z.B. ätherische Öle, Emulgatoren, Zucker/Zuckeraustausch- oder Zuckerersatzstoffe, andere Mineralien, Verdickungsmittel, Trennmittel, Puffer/Pufferungssubstanzen, Stabilisatoren wie Traganth, Gummi arabicum, Pektin, Gelatine, Carageen, Säuerungsmittel, Antioxidationsmittel, oder Mischungen aus 2 oder mehreren Vertretern dieser Substanzen. Weiterhin möglich sind Lösungsvermittler.

### Verwendung

Die erhaltenen Hydroxycarbonsäure-stabilisierten Magnesiumcitratlösungen können in unterschiedlichen galenischen Formen verwendet werden. Dazu können geeignete Zusatzstoffe wie beschrieben eingesetzt werden. Diese werden vorzugsweise ausgewählt aus Aromen/Geschmacksstoffen, insbesondere ätherischen Ölen, Vitaminen, anderen Mineralien, sowie galenischen Hilfsstoffen, ausgewählt aus Zuckern, Zuckeraustauschstoffen, Zuckerersatzstoffen (Süßungsmitteln), Säuerungsmitteln, Lösungsvermittlern wie z. B. Wasser, Glykol, Glycerin, Lecithin, Verdickungsmitteln, Farbstoffen oder Konservierungsmitteln oder Kombinationen hiervon.

Die erfindungsgemäße Hydroxycarbonsäure-stabilisierte Trimagnesiumdicitratlösung eignet sich besonders als solche zur Behebung eines Magnesiummangels in Form von Lebensmitteln, oder auch Arzneimitteln für besondere medizinische Zwecke. Sie kann aber auch verwendet werden zur Vorbeugung von Mangelsituationen bei Gesunden als Nahrungsergänzung zur Supplementierung der täglichen Ernährung, bzw. zur Anreicherung von Lebensmitteln wie Säften, Limonaden, oder Pudding, Quark, u. ä. mit Magnesium, Aufgrund der hohen WirkstoffKonzentration kann dabei die absolute Einsatzmenge sehr niedrig gehalten werden.

Im Allgemeinen werden hiermit Magnesiummengen erzielt, die mindestens 15 % der RDA (also 15 % der recommended daily bzw. dietary allowance) enthält. Dies können vor allem Zubereitungen mit 400 mg 25 mL⁻¹ oder 400 mg 25 mL⁻¹ Magnesium (also mit 16 g(Mg²⁺) L⁻¹ oder 40 g(Mg²⁺) L⁻¹ an reinem Magnesium) sein. Dagegen wird mit handelsüblichem Magnesium-Zubereitungen bisher nur maximal ein Gehalt von 300 mg 25 mL⁻¹ Magnesium (also 12 g(Mg²⁺) L⁻¹ an reinem Magnesium) erhalten.

Besonders bevorzugt als galenische Zubereitungsform ist ein gebrauchsfertiges Getränk, insbesondere ein wässriges Getränk, z.B. vorzugsweise mit Konservierungsmitteln, Farbstoffen, Geschmacksstoffen oder Mischungen hiervon, ein Fruchtsaftgetränk (z.B. ein wässriges Getränk mit Aroma(=Geschmacks)-stoffen versetzt), oder ein Sirup (Getränk mit Verdickungsmitteln als galenische Hilfsstoffe) oder ein Getränkekonzentrat, z.B. in Dosiereinheiten von vor allem je 25 mL oder 10 mL mit einem Magnesiumgehalt von jeweils 400 mg.

Im vorliegenden Fall werden insbesondere flüssige Darreichungsformen bevorzugt.

Durch die hohe Anreicherung dieser galenischen Formen mit Magnesium ist es möglich, selbst bei sehr kleinen Verzehrmengen eine hohe mineralische Versorgung zu erreichen.

Die anmeldungsgemäßen Zubereitungen sind z.B. einsetzbar bei Säugetieren, insbesondere bei Menschen, besonders bevorzugt bei Menschen mit einem erhöhten Bedarf an Magnesium wie Sportler, ältere Menschen, Kleinkinder, Kranke, Mangelernährte oder Menschen mit allgemeinem Magnesiummangel. Sie eignen sich als Arzneimittel, diätetische Lebensmittel für besondere medizinische Zwecke, bzw. als Nahrungsergänzungsmittel oder Lebensmittel direkt oder zur Anreicherung von Lebensmitteln wie z.B. "Functional Food".

Die Menge an verabreichtem bzw. verzehrtem erfindungsgemäßen Hydroxycarbonsäure-stabilisierten Magnesiumcitrats richtet sich nach der jeweiligen Personengruppe und/oder dem akuten oder allgemein empfohlenen Bedarf.

Insbesondere ist die übliche Menge in den beschriebenen galenischen Formen so berechnet, dass mit einer geringen Dosierung ca. 1 % bis ca. 140 %, oder bevorzugt ca. 15 % bis ca. 133 % und vor allem ca. 50 % bis ca. 125 % des Tagesbedarfes an Magnesium abgedeckt werden können. Dazu eignen sich vor allem flüssige Darreichungsformen wie wässrige Zubereitungen, z.B. mit Aroma(=Geschmacksstoffen) angereicherte Dosierungen von 10 mL bis 250 mL, insbesondere 10 mL bis 25 mL, jeweils besonders bevorzugt à 400 mg Magnesium.

Es können dazu auch Mehrfachpackungen zur Verfügung gestellt werden, z.B. eine Packung mit 10 Einzelpackungen ä 10 mL oder eine Packung mit 5 Einzelpackungen ä 25 mL, vor allem jeweils mit einem Gehalt an gelöstem Magnesium von 400 mg in Wasser (bevorzugt weiterhin umfassend weitere Wirkstoffe wie Vitamine, Mineralien und/oder Spurenelemente, Aromen, Konservierungsmittel, Süßungsmittel, Geschmacksstoffe und übliche Hilfs- und Zusatzstoffe).

In einer anderen Ausführungsform können derartige Zubereitungen auch als pharmazeutische Zubereitungen eingesetzt werden, und zwar vor allem in Form der beschriebenen wässrigen Lösungen, aber auch Sirupen oder wässrig-alkoholhaltigen Lösungen durch Zusatz jeweils geeigneter gewünschter Zusatzstoffe bzw. pharmazeutisch üblicher, zulässiger Zusatz-/Hilfsstoffe.

In einer bevorzugten Ausführungsform werden die beschriebenen Zubereitungsformen, insbesondere wässrige Zubereitungen, Sirupe und andere Trinklösungen eingesetzt zur Behandlung von Magnesiummangelzuständen, z.B. gekennzeichnet durch Muskelkrämpfe oder zur Beseitigung übermäßiger Magnesiumverluste, z.B. durch intensive sportliche Betätigung.

### Beispiele

Die Erfindung wir anhand folgender Beispiele erläutert:
Beispiel 1 Zunächst wurde Magnesiumcitrat hergestellt durch Lösen von 216,15 g (1,03 Mol) Citronensäure als Pulver in 500 g Wasser. Die Zubereitung wurde vor Zugabe von Magnesiumoxid erwärmt auf 70°C. Dann wurde unter Rühren 64,66 g (1,54 Mol) Magnesiumoxid hinzugefügt. Nach vollständiger Ausbildung einer Lösung unter Rühren wurde nach Abkühlung auf Raumtemperatur 119,69 g (1,16 Mol) Milchsäure (88 Gew.-%ige Lösung) hinzugefügt und gerührt. Anschließend wurde die entstandene Lösung auf ein Liter mit Wasser aufgefüllt. Es wird eine klare Lösung mit Milchsäure-stabilisierter Magnesiumcitratlösung mit einem Magnesiumgehalt von 40 g(Mg²⁺) L⁻¹ erhalten.
Beispiel 2 Zunächst wurde Magnesiumcitrat hergestellt durch Lösen von 216,15 g (1,03 Mol) Citronensäure als Pulver in 500 g Wasser. Die Zubereitung wurde vor Zugabe von Magnesiumoxid erwärmt auf 70°C. Dann wurde unter Rühren 64,66 g (1,54 Mol) Magnesiumoxid hinzugefügt. Nach vollständiger Ausbildung einer Lösung unter Rühren wurde nach Abkühlung auf Raumtemperatur 79,45 g (0,77 Mol) Milchsäure (88 Gew.-%ige Lösung) und 103,25 g (0,77 Mol) Äpfelsäure hinzugefügt und gerührt. Anschließend wurde die entstandene Lösung auf ein Liter mit Wasser aufgefüllt, Es wird eine klare Lösung mit Milch-/Äpfelsäure-stabilisierter Magnesiumcitratlösung mit einem Magnesiumgehalt von 40 g(Mg²⁺) L⁻¹ erhalten.
Beispiel 3 Wie in Beispiel 1 und 2 geschildert, wurden unterschiedlich konzentrierte Hydroxycarbonsäure- stabilisierte Magnesiumcitratlösungen mit Milchsäure, Äpfelsäure und Mischungen hiervon hergestellt und die Löslichkeit des Magnesiumgehaltes bestimmt durch komplexometrische Titration. Die Werte ergeben sich jeweils aus einer Dreifachbestimmung jeder einzelner Probe. Die Ergebnisse sind in nachfolgender Tabelle 1 sowie in Abbildung 1 dargestellt.

**Tabelle 1**

| Versuch | Mg-Konzentration | x* | y* | l* | m* | Mg-Konzentration (Messung) | |
|---|---|---|---|---|---|---|---|
| | (g(Mg²⁺)L⁻¹) | (-) | (-) | (-) | (-) | (g(Mg²⁺) L⁻¹) | (±) |
| 3.1 | 50,00 | 3 | 2 | 0,750 | 0,000 | 24,51 | 0,25 |
| 3.1 | 50,00 | 3 | 2 | 1,500 | 0,000 | 32,93 | 0,76 |
| 3.1 | 50,00 | 3 | 2 | 2,250 | 0,000 | 42,53 | 1,17 |
| 3.1 | 50,00 | 3 | 2 | 3,000 | 0,000 | 41,94 | 0,74 |
| 3.1 | 50,00 | 3 | 2 | 3,750 | 0,000 | 37,96 | 1,52 |
| 3.2 | 50,00 | 3 | 2 | 0,000 | 0,750 | 31,88 | 1,03 |
| 3.2 | 50,00 | 3 | 2 | 0,000 | 1,500 | 44,36 | 0,44 |
| 3.2 | 50,00 | 3 | 2 | 0,000 | 2,250 | 47,76 | 0,64 |
| 3.2 | 50,00 | 3 | 2 | 0,000 | 3,000 | 47,92 | 0,07 |
| 3.2 | 50,00 | 3 | 2 | 0,000 | 3,750 | 41,44 | 1,93 |
| 3.3 | 50,00 | 3 | 2 | 0,375 | 0,375 | 30,91 | 1,18 |
| 3.3 | 50,00 | 3 | 2 | 0,750 | 0,750 | 44,52 | 0,49 |
| 3.3 | 50,00 | 3 | 2 | 1,125 | 1,125 | 48,12 | 1,75 |
| 3.3 | 50,00 | 3 | 2 | 1,500 | 1,500 | 47,48 | 0,62 |
| 3.3 | 50,00 | 3 | 2 | 1,875 | 1,875 | 46,99 | 1,28 |
| Mittelwert Referenz | 50,00 | 3 | 2 | 0,000 | 0,000 | 8,08 | 4,83 |

| | | | | | | | |
|---|---|---|---|---|---|---|---|
| *(x = n(Mg²⁺); y = n(Citronensäure); l = n(Milchsäure); m = n(Äpfelsäure) | | | | | | | |

## Patentansprüche

1. Verfahren zur Herstellung eines flüssigen Produktes mit einem Gehalt an stabilisiertem Magnesium in hoher Bioverfügbarkeit, **gekennzeichnet durch** folgende Schritte:
i) Lösen geeigneter vorbestimmter Mengen (mol) an Citronensäure in einem geeigneten Lösungsmittel wie Wasser, bevorzugt unter Rühren und/oder bei Temperaturen von 20 °C bis 99 °C;
ii) Zugabe einer molaren Menge, bezogen auf die Citronensäure, einer oder mehrerer, vor allem anorganischer Magnesium(ausgangs)-verbindungen, ausgewählt aus Magnesiumsalzen, und unter Rühren;
iii) Zugabe einer molaren (stöchiometrischen) Menge, bezogen auf die Magnesium(ausgangs)-verbindung, an Hydroxycarbonsäure;
iv) Gewinnung des so erhaltenen Produktes mit einem hohen Gehalt an bioverfügbarem, gelösten Magnesium in an sich bekannter Weise, wobei das stabilisierte Magnesium als Hydroxycarbonsäure-stabilisierte Magnesiumcitratverbindung der allgemeinen Formel (I)
Mgₓ(C₆H₅O₇)_{y}(HCS)_{z}*(14-z)•H₂O (I)
vorliegt, worin x = 3, y = 2, z = 0,25 - 4,00, Mg = Magnesium-Ion, und (C₆H₅O₇)= Citrat-Ion, HCS= Hydroxycarbonsäure bedeuten.

2. Verfahren nach Anspruch 1, **dadurch gekennzeichnet, dass** die Umsetzung in Schritt ii) bei einer Temperatur von 70 °C bis 95 °C erfolgt.

3. Verfahren nach Anspruch 1 oder 2, **dadurch gekennzeichnet, dass** als Magnesium(ausgangs)-verbindung in Schritt ii) Magnesiumoxid, Magnesiumcarbonat, Magnesiumhydroxid oder Magnesiumchlorid hinzugefügt wird.

4. Verfahren nach einem der Ansprüche 1 bis 3, **dadurch gekennzeichnet, dass** die Hydroxycarbonsäure gemäß Schritt iii) ausgewählt ist aus Glykolsäure, Milchsäure, Äpfelsäure, Weinsäure oder auch Gluconsäure, Glucuronsäure, Glycerinsäure, Xylonsäure, Galacturonsäure, Iduronsäure, Mucinsäure, Glucarsäure Chinasäure, oder Mischungen hiervon.

5. Verfahren nach Anspruch 4, **dadurch gekennzeichnet, dass** als Hydroxycarbonsäure Milchsäure, Äpfelsäure oder Mischungen hiervon ausgewählt sind.

6. Verfahren nach einem der Ansprüche 1 bis 5, **dadurch gekennzeichnet, dass** nach Schritt iii) vor Schritt iv) geeignete/gewünschte übliche Hilfs- oder Zusatzstoffe, ausgewählt aus Aromen/Geschmacksstoffen, Vitaminen, Mineralien, galenischen Hilfsstoffen, ausgewählt aus Zuckern, Zuckeraustauschstoffen, Zuckerersatzstoffen Säuerungsmitteln, Lösungsvermittlern, Verdickungsmitteln, Farbstoffen oder Konservierungsmitteln oder Kombinationen hiervon hinzugefügt werden.

7. Produkt in Form einer flüssigen Zubereitung, umfassend Wasser als Lösungsmittel und einen Gehalt an stabilisiertem Magnesium in hoher Bioverfügbarkeit, **dadurch gekennzeichnet, dass** das stabilisierte Magnesium in einer Konzentration von ≥ 20 g(Mg²⁺) L⁻¹ im Lösungsmittel als Hydroxycarbonsäure-stabilisierte Trimagnesiumdicitrat der allgemeinen Formel (I)
Mgₓ(C₆H₅O₇)_{y}(HCS)_{z}(14-z)H₂O (I)
vorliegt, worin x = 3, y = 2, z = 0,25 - 4,00, C₆H₅O₇ das Citrat-Ion, und Mg das Magnesium-Ion, HCS= Hydroxycarbonsäure bedeuten.

8. Produkt gemäß Anspruch 7, **dadurch gekennzeichnet, dass** als Hydroxycarbonsäure Glykolsäure, Milchsäure, Äpfelsäure, Weinsäure oder auch Gluconsäure, Glucuronsäure, Glycerinsäure, Xylonsäure, Galacturonsäure, Iduronsäure, Mucinsäure, Glucarsäure Chinasäure, oder Mischungen hiervon vorliegen.

9. Produkt gemäß einem der Ansprüche 7 oder 8, **dadurch gekennzeichnet, dass** als Hydroxycarbonsäure Milchsäure, Äpfelsäure oder Mischungen hiervon vorliegen.

10. Produkt gemäß einem der Ansprüche 7 bis 9, umfassend Wasser sowie eine Hydroxycarbonsäure-stabilisierte Trimagnesiumdicitratlösung gemäß Formel (I), wobei das stabilisierte Magnesium in einer Konzentration von 45 g(Mg²⁺) L⁻¹ vorliegt.

11. Produkt in Form einer flüssigen Zubereitung, gemäß einem der Ansprüche 7 bis 10, **dadurch gekennzeichnet, dass** das stabilisierte Magnesium ausgewählt ist aus einer Trimagnesiumdicitratverbindung der allgemeinen Formel
Mgₓ(C₆H₅O₇)_{y}(C₄H₆O₅)_{z}*(14-z)•H₂O (II)
(x = 3, y = 2, z = 0,25 - 3,75)
Mgₓ(C₆H₅O₇)_{y}*[(C₃H₆O₃)ₗ(C₄H₆O₅)ₘ]_{z} *(14-z)•H₂O (III)
(x = 3, y = 2, z = I + m = 0,25 - 3,75)
oder
Mgₓ(C₆H₅O₇)_{y}*[(C₃H₆O₃)ₗ(C₄H₆O₅)ₘ]_{z}*(14-z)•H₂O (IV)
(x = 3, y = 2, z = l + m = 0,25 - 3,75).

12. Produkt gemäß einem der Ansprüche 7 bis 11, **dadurch gekennzeichnet, dass** es in Form eines Nahrungsergänzungsmittels, Medizinproduktes oder Lebensmittels vorliegt.

13. Produkt gemäß Anspruch 12, **dadurch gekennzeichnet, dass** es als gebrauchsfertiges wässriges Getränk, als wässriges Getränk mit Aroma(=Geschmacks)-stoffen (Fruchtsaftgetränk), als wässriges Getränkekonzentrat oder als wässriges verdicktes Getränk (Sirup), in Dosiereinheiten von je 25 mL oder 10 mL mit einem Magnesiumgehalt von jeweils 400 mg vorliegt.

14. Produkt gemäß einem der Ansprüche 7 bis 12 oder hergestellt nach einem der Ansprüche 1 bis 6 zur (nicht therapeutischen) Verwendung als Nahrungsergänzungsmittel, Lebensmittel, Medizinprodukt (für besondere nicht therapeutische Zwecke) oder als angereichertes Lebensmittel (Functional Food) oder zur Herstellung eines Nahrungsergänzungsmittels, Medizinproduktes oder Lebensmittels.

15. Produkt gemäß einem der Ansprüche 7-11 oder hergestellt nach einem der Ansprüche 1 bis 6 zur Verwendung als Arzneimittel oder zur Herstellung eines Arzneimittels.
